# EUROPÄISCHE PATENTANMELDUNG

(11) **EP 1 157 668 A1**
(43) Veröffentlichungstag der Anmeldung: **28.11.2001**
(21) Anmeldenummer: 00110798.6
(22) Anmeldetag: 20.05.2000
(51) Int. Cl.: A61B 18/14

(54) **Elektrochirugische Vorrichtung zur Vernarbung eines Sphinkter-Muskels**

(71) Anmelder: Curative AG Innovations to cure, 01307 Dresden (DE)
(72) Erfinder: Holger, Friedrich Dr., 01277 Dresden (DE); Petersen, Sven Dr., 21039 Börnsen (DE)
(74) Vertreter: von Kirschbaum, Alexander, Dipl.-Ing.

(57) **Zusammenfassung**

Eine Vorrichtung zur Vernarbung des Sphinkter-Muskels weist einen flexiblen Schlauch (10) auf in dem Energieleitungen (12) vorgesehen sind. Die Energieleitungen (12) sind mit in Längsrichtung des Schlauchs (10) verschiebbaren Elektroden (14) verbunden. Die Elektroden (14) sind abgewinkelt und elastisch verformbar, so dass sie aus einem Behandlungszustand in dem sie zeitlich über dem Schlauch hervorstehen durch Betätigen einer Betätigungsvorrichtung (16) in den Schlauch (10) zurückgezogen werden können, wobei die Elektrode (14) verformt werden. Durch die Vorrichtung kann an den Sphinkter-Muskel eine hochfrequente Strom erzeugende Energiequelle angelegt werden. Hierdurch werden an dem Sphinkter-Muskel Narben erzeugt, durch die der Durchmesser des Sphinkter-Muskels abnimmt. Hierdurch kann die Gastro-ösophageale Reflux-Krankheit behandelt werden.

## Beschreibung

Die Erfindung betrifft eine Vorrichtung zur Vernarbung eines Sphinkter-Muskels, insbesondere des ösophagealen Sphinkter-Muskels. Bei dem ösophagealen Sphinkter-Muskel handelt es sich um den zwischen dem Magen und der Speiseröhre angeordneten Schließmuskel.

Wenn der Sphinkter-Muskel den Übergang zwischen Magen und Speiseröhre nicht vollständig verschließt, kann es zur so genannten Gastro-ösophagealen Relux-Krankheit kommen. Bei dieser tritt saurer Mageninhalt in die Speiseröhre ein und führt dort zu Entzündungen der Speiseröhren-Schleimhaut. Dies kann zu äußerst schmerzvollen und bösartigen Veränderungen der Speiseröhren-Schleimhaut führen.
kann zu äußerst schmerzvollen und bösartigen Veränderungen der Speiseröhren-Schleimhaut führen.

Um die Schließfunktion des Sphinkter-Muskels zu verbessern, kann zur Verringerung des Durchmessers des Sphinkter-Muskels eine narbige Verschrumpfung des Muskels hervorgerufen werden. Es handelt sich hierbei um eine bewusst herbeigeführte Narbenbildung. Diese kann durch Nadeln hervorgerufen werden. Hierzu werden mit Hilfe eines Katheters, Nadeln in die Speiseröhre eingeführt und im Bereich des Sphinkter-Muskels durch einen Ballonmechanismus in den Muskel eingestochen. Das gezielte Verletzen und anschließende Heilen der Muskeloberfläche führt hierbei zur Narbenbildung. Das Erzeugen von Narben durch Nadeln ist jedoch nur schwer durchzuführen, insbesondere sind derartige Geräte schwer zu handhaben. Ferner ist es schwierig durch Nadelstiche eine definierte Narbenbildung und somit eine definierte Verringerung des Durchmessers der Muskel-Öffnung hervorzurufen.

Aufgabe der Erfindung ist es eine Vorrichtung zur Vernarbung des Sphinkter-Muskels zu schaffen, die einfach zu handhaben ist und mit der eine definierte Vernarbung erzielt werden kann.

Die Lösung der Aufgabe erfolgt erfindungsgemäß durch die Merkmale des Anspruchs 1.

Die Erfindung beruht auf der Erkenntnis eine Vernarbung durch Strom-Applikation hervorzurufen. Hier werden die Narben dadurch erzeugt, dass über eine Elektrode Strom an das Gewebe angelegt wird. Durch die Energieabgabe wird eine Verkochung des Gewebes bewirkt. Die verkochten Anteile schrumpfen während der Heilphase narbig ein. Hierbei ist die Stärke der Vernarbung erheblich besser steuerbar.

Die erfindungsgemäße Vorrichtung zur Vernarbung des Sphinkter-Muskels weist mindestens eine in einem flexiblen Schlauch angeordnete Energieleitung und eine mit der Energieleitung verbundene ausfahrbare Elektrode auf. In einer ersten Position ist die Elektrode innerhalb des Schlauchs angeordnet. In dieser Position kann der Schlauch zusammen mit der in ihm angeordneten Energieleitung und der Elektrode durch die Speiseröhre eingeführt werden, bis das Ende des Schlauchs im Bereich des Sphinkter-Muskels ist. In einer zweiten Position steht das freie Ende der Elektrode über einem Ende des Schlauches vor und ist somit ausgefahren. Da in der zweiten Position das Elektrodenende frei, d. h. außerhalb des Schlauchs angeordnet ist, kann die Elektrode in den. Sphinkter-Muskel eindringen und mit Energie beaufschlagt werden, so dass durch leichtes Verletzen und anschließendes Heilen des Muskels eine definierte Narbenbildung erfolgt.

Zur Überführung der Vorrichtung aus der ersten Position in die zweite Position ist die Elektrode in dem Schlauch vorzugsweise in Längsrichtung verschiebbar. Somit kann auf einfache Weise nach dem Einführen des Schlauchs in die Speiseröhre die Vorrichtung beispielsweise durch einen Bautenzug oder dergleichen in die zweite Position übergeführt werden. Durch die Relativbewegung zwischen dem Schlauch und der Elektrode tritt diese aus dem Schlauchende hervor. Die erfindungsgemäße Vorrichtung ist somit äußerst einfach zu handhaben. Der Schlauch dient herbei als Außenführung. Ferner kann die Vorrichtung in ihrer ersten Position sicher durch die Speiseröhre eingeführt werden, ohne durch vorstehende Elektrodenenden Verletzungen in der Speiseröhre hervorzurufen. Der Schlauch dient somit beim Einführen der Vorrichtung als Schutz.

Die Elektrode steht in der zweiten Position vorzugsweise seitlich über die Außenseite des Schlauches hervor. Hierdurch ist eine einfachere Behandlung des Sphinkter-Muskels möglich. Insbesondere kann durch das seitliche Hervorstehen der Elektroden die Innenseite des Sphinkter-Muskels durch die der Durchmesser des Sphinkter-Muskels definiert wird, gut behandelt bzw. vernarbt werden. Hierzu kann an dem Ende des Schlauchs, das bei der Behandlung im Bereich des Sphinkter-Muskels angeordnet ist eine Führung vorgehen sein. Durch die Führung werden beispielsweise flexible Elektroden bei Überführen des Schlauchs von der ersten Position in die zweite Position radial nach außen geführt, so dass sie seitlich über 'die Außenwände des Schlauchs hervorstehen.

Bei einer bevorzugten Ausführungsform sind die Elektroden abgewinkelt und elastisch verformbar ausgebildet. Die Elektroden sind somit im unverformten Zustand abgewinkelt. Dies hat zur Folge, dass die Elektrode in der zweiten Position automatisch beim Ausfahren aus dem Schlauch seitlich ausgespreizt wird, so dass sie seitlich über die Außenseite des Schlauches vorsteht. Eine Führung kann hierbei entfallen. Da die Elektrode elastisch verformbar ist, kann sie beim Überführen aus der zweiten Position in die erste Position, d. h. beim Rückziehen der Elektrode in den Schlauch elastisch verformt werden und ist in der ersten Position somit innerhalb des Schlauchs angeordnet.

Die Elektrode und die Energieleitung sind vorzugsweise einstückig, so dass durch Verschieben der Energieleitung in Längsrichtung relativ zum Schlauch ein Verschieben der Elektrode stattfindet. Es ist somit nicht erforderlich einen zusätzlichen Bautenzug innerhalb des Schlauchs vorzusehen. Das Verschieben der Elektrode kann unmittelbar durch Verschieben der Energieleitung in dem Schlauch erfolgen. Die elastisch verformbare Elektrode ist vorzugsweise aus einem Material, das einen Memory-Effekt aufweist. Es handelt sich hierbei vorzugsweise um Nitinol, Platin, Kupfer oder einem anderen Edelmetall.

Auf Grund der elastischen Verformbarkeit der Elektrode ist ein zusätzliches Expansions-Hilfsmittel oder Ähnliches nicht erforderlich.

Vorzugsweise weist die Vorrichtung zur Behandlung des Sphinkter-Muskels eine Betätigungsvorrichtung zur Längsverschiebung der Energieleitung relativ zum Schlauch auf. Die Betätigungsvorrichtung besteht aus zwei Betätigungselementen, wobei ein Betätigungselement mit der Elektrode und das andere Betätigungselement mit dem Schlauch verbunden ist. Wenn die Elektrode und die Energieleitung einstückig ausgebildet sind, kann das eine Betätigungselement direkt mit der Energieleitung verbunden sein. So dass durch Betätigen der beiden Betätigungselemente die Energieleitung und damit gleichzeitig die Elektrode relativ zum Schlauch verschoben wird.

Die Elektrode ist über die Energieleitung vorzugsweise mit einer Energiequelle verbunden, die hochfrequenten Strom erzeugt. Als Energiequelle kann ein Radiofrequenzgenerator verwendet werden, der eine Leistung von bis zu 100 W aufweist. Der Elektrode kann somit Strom im Radiofrequenzbereich zugeführt werden.

Bei einer besonders bevorzugten Ausführungsform sind in dem Schlauch mehrere Elektroden vorgesehen. Insbesondere sind mindestens 4, vorzugsweise 8 und insbesondere 16 Elektroden vorgesehen. Wenn als Elektroden elastisch verformbare abgewinkelte Elektroden vorgesehen werden, sind diese vorzugsweise sternförmig angeordnet. Dies hat zur Folge, dass im Behandlungszustand mehrere Elektroden sternförmig seitlich über die Schlauchaußenseite vorstehen. Die Elektroden sind hierbei vorzugsweise regelmäßig am Umfang des Schlauches verteilt, so dass eine gleichmäßige Narbenbildung der Innenseite des Sphinkter-Muskels hervorgerufen werden kann. Das Vorsehen mehrerer Elektroden hat ferner den Vorteil, dass sich die Elektroden gegeneinander abstützen.

Der Winkel der abgewinkelten Elektroden beträgt in unverformtem Zustand vorzugsweise 45° bis 135°. Insbesondere ist ein Winkel von 80° - 100° vorteilhaft, da somit bei exakt in die Speiseröhre eingeführten Schlauch sichergestellt ist, dass die Innenseite des Sphinkter-Muskels mit Strom behandelt wird.

Vorzugsweise sind an den Elektrodenenden Temperatursensoren vorgesehen, von denen die Umgebungstemperatur des zu behandelnden Gewebes gemessen wird. Die Energiezufuhr wird über die bei der Behandlung auftretende Wärme gesteuert, so dass bei einer oberen Grenztemperatur die Energiezufuhr automatisch abgeschaltet wird. In Verbindung mit dem Radiofrequenzgenerator handelt es sich somit um eine temperaturgesteuerte Radiofrequenzabgabe.

Da das Einführung eines Schlauches in die Speiseröhre für den Patienten äußerst unangenehm ist, werden vorzugsweise Elektroden mit einem Durchmesser verwendet, der kleiner als 1 mm, insbesondere kleiner als 0,5 mm ist. Bei einem Elektrodendurchmesser von etwa 0,3 mm können in einem Schlauch mit einem Innendurchmesser von etwa 3 mm 16 Elektroden angeordnet werden.

Vorzugsweise wird die erfindungsgemäße Vorrichtung zusammen mit einem Gastroskop verwendet, wobei die Vorrichtung durch den Arbeitskanal des Gastroskops eingeführt wird. Dies hat den Vorteil, dass der Arzt mit Hilfe des Gastroskops genau beobachten kann, an welcher Stelle sich die Elektrodenenden befinden. Es ist somit eine äußerst genaue Zufuhr von Strom zu dem Sphinkter-Muskel möglich. Die Lage der Vernarbung an dem Sphinkter-Muskel kann somit genau gesteuert werden.

Der Patient wird bei der Vernarbung üblicherweise mit Masse verbunden. Dies erfolgt beispielsweise über eine am Rücken des Patienten vorgesehene Gegenelektrode. Ebenso kann eine oder mehrere der an dem Sphinkter-Muskel anliegenden Elektroden als Gegenelektrode verwendet werden.

Nachfolgend wird die Erfindung anhand einer bevorzugten Ausführungsform unter Bezugnahme auf die anliegenden Zeichnungen näher erläutert.

Es zeigen:
- Fig. 1: eine schematische Ansicht der erfindungsgemäßen Vorrichtung in der ersten Position,
- Fig. 2: eine schematische Darstellung der erfindungsgemäßen Vorrichtung in der zweiten Position,
- Fig. 3: eine schematische Ansicht entlang der Linie III - III in Fig. 2 und
- Fig. 4: eine schematische Ansicht in Richtung des Pfeils IV in Fig. 2.

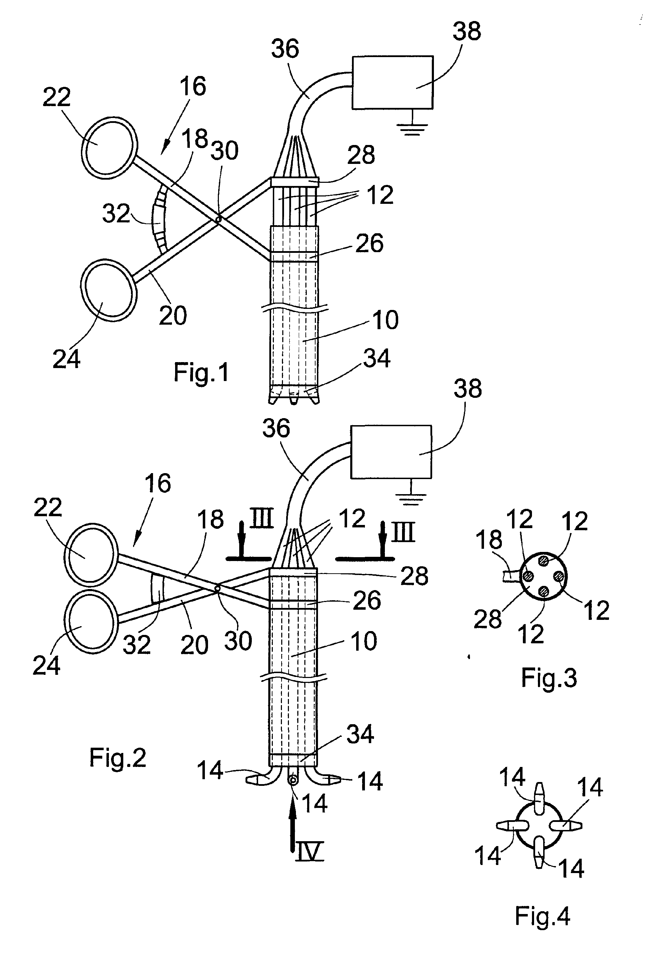

In einem flexiblen langgestreckten Schlauch 10, der in die Speiseröhre eingeführt werden kann, sind in dem dargestellten Ausführungsbeispiel vier Energieleitungen 12 vorgesehen. Die Energieleitungen 12 erstrecken sich über die gesamte Länge des Schlauchs 10 und sind gleichmäßig um den Umfang des Schlauchs verteilt. An dem in Fign. 1 und 2 unteren Ende der Energieleitungen 12 sind die Energieleitungen 12 mit abgewinkelten Elektroden 14 verbunden. Die Elektroden bestehen aus einem elastischen Material und weisen in unverformtem Zustand einen Winkel von etwa 90° auf, so dass sie in dem in Fig. 2 dargestellten Behandlungszustand sternförmig über die Außenseite des Schlauchs 10 hervorstehen (Fig.4).

Um die Vorrichtung aus der in Fig. 1 dargestellten ersten Position in die in Fig. 2 dargestellte zweite Position überzuführen, ist eine Betätigungsvorrichtung 16 vorgesehen. Die Betätigungsvorrichtung 16 weist zwei Betätigungselemente 18, 20 auf, die scherenartig miteinander verbunden sind. Die beiden Betätigungselemente 18, 20 weisen jeweils einen Griffteil 22, 24 auf, die entsprechend eines Scherengriffes ausgebildet sind. Das Betätigungselement 18 ist über einen Haltering 26 fest mit dem Schlauch 10 verbunden. Das Betätigungselement 20 ist über ein Halteelement 28 fest mit den Energieleitungen 12 verbunden. Das Halteelement 28 ist vorzugsweise kreisförmig und weist mehrere Durchgangsbohrungen auf, in denen die Energieleitungen gehalten sind. Zwischen den beiden um ein Gelenk 30 miteinander verschränkbaren Betätigungselementen 18, 20 ist eine Druckfeder 32 vorgesehen. Durch die Druckfeder 32 wird die Betätigungsvorrichtung 16 in die in Fig. 1 dargestellte Stellung, d. h. in die erste Position gebracht. Durch Zusammendrücken der Druckfeder 32, d. h. durch Betätigen der Betätigungsvorrichtung 16 wird das Halteelement 28 in Richtung des Schlauchs 10 bewegt. Dadurch werden die Energieleitungen 12 in den Fign. 1 und 2 nach unten bewegt, so dass die Elektroden 14 aus dem unteren Ende des Schlauchs 10 austreten.

Das zurückziehen der Elektroden 14 in den Schlauch 10 erfolgt durch einfaches Loslassen der Betätigungsvorrichtung 16, da hierbei durch die Druckfeder 32 die beiden Betätigungselemente 18, 20 automatisch auseinander bewegt werden. Um hierbei ein Beschädigen des Endes des Schlauchs 10 zu vermeiden und sicherzustellen, dass die Elektroden elastisch verformt und vollständig in den Schlauch eingezogen werden, ist an dem Ende des Schlauchs 10 ein Versteifungsring 34 vorgesehen.

Die Energieleitungen 12 sind über eine Leitung 36 mit einer Energiequelle 38 verbunden. Von der Energiequelle 38 wird ein hochfrequenter Strom erzeugt, der über die Leitung 36 die einzelnen Energieleitungen 12 und die Elektroden 14 in dem Behandlungszustand an dem Sphinkter-Muskel angelegt werden. Hierzu ist an der Energiequelle 38 oder in der Leitung 36 bzw. in einem gegebenenfalls zusätzlich vorgesehenen Steuergerät ein entsprechender Schalter vorgesehen. Mit Hilfe des Steuergeräts kann ferner die Stärke des Stroms die Frequenz sowie die Spannung eingestellt werden.

## Patentansprüche

1. Vorrichtung zur Vernarbung des Sphinkter-Muskels,
mit mindestens einer in einem flexiblen Schlauch (10) angeordneten Energieleitung (12) und
einer mit der Energieleitung (12) verbundenen, aus dem Schlauch (10) ausfahrbaren Elektrode (14).

2. Vorrichtung nach Anspruch 1, **dadurch gekennzeichnet, dass** die Elektrode (14) in dem Schlauch (10) in Längsrichtung verschiebbar und elastisch nach außen vorgespannt ist, so dass sie beim Ausfahren seitlich ausgespreizt wird.

3. Vorrichtung nach Anspruch 1 oder 2, **dadurch gekennzeichnet, dass** eine Betätigungsvorrichtung (16) mit zwei Betätigungselementen (18, 20) zur Längsverschiebung der Elektrode (14) relativ zum Schlauch (10) vorgesehen ist, deren eines Betätigungselement (20) mit der Elektrode (14) und deren anderes Betätigungselement (18) mit dem Schlauch (10) verbunden ist.

4. Vorrichtung nach Anspruch 3, **dadurch gekennzeichnet, dass** die beiden Betätigungselemente scherenartig miteinander verbunden sind.

5. Vorrichtung nach einem der Ansprüche 1 - 4, **dadurch gekennzeichnet, dass** die Energieleitung (12) mit einer hochfrequenten Strom erzeugenden Energiequelle (38) verbunden ist.

6. Vorrichtung nach einem der Ansprüche 1 - 5, **dadurch gekennzeichnet, dass** die Elektrode (14) mit einem Temperatursensor zur Steuerung der der Elektrode zuzuführenden Energie verbunden ist.

7. Vorrichtung nach einem der Ansprüche 1 - 6, **dadurch gekennzeichnet, dass** mindestens vier, vorzugsweise mindestens acht und insbesondere mindestens sechszehn Elektroden vorgesehen sind.

8. Vorrichtung nach einem der Ansprüche 2 - 7, **dadurch gekennzeichnet, dass** der Winkel der Elektrode in unverformtem Zustand 45° - 135°, vorzugsweise 80° - 100° beträgt.

9. Vorrichtung nach einem der Ansprüche 1 - 8, **dadurch gekennzeichnet, dass** der Elektrodendurchmesser kleiner als 1 mm, insbesondere kleiner als 0,5 mm ist.

10. Gastroskop mit einer in einem Arbeitskanal des Gastroskops angeordneten Vorrichtung zur Vernarbung des Sphinkter-Muskels nach einem der Ansprüche 1 - 9.

11. Verwendung der Vorrichtung nach einem der Ansprüche 1 - 9, in Verbindung mit einem Gastroskop, wobei die Vorrichtung durch einen Arbeitskanal des Gastroskops eingeführt wird.
